Europäisches Patentamt

European Patent Office

Office européen des brevets

(11) Numéro de publication: **0 312 453 B1**

## (12) FASCICULE DE BREVET EUROPEEN

(45) Date de publication de fascicule du brevet: **12.01.94**   (51) Int. Cl.5: **C12P 7/40**

(21) Numéro de dépôt: **88402583.4**

(22) Date de dépôt: **12.10.88**

(54) **Procédé de production d'acide pyruvique par fermentation.**

(30) Priorité: **15.10.87 FR 8714248**

(43) Date de publication de la demande:
**19.04.89 Bulletin 89/16**

(45) Mention de la délivrance du brevet:
**12.01.94 Bulletin 94/02**

(84) Etats contractants désignés:
**DE ES IT**

(56) Documents cités:
**FR-A- 2 277 890**

**CHEMICAL ABSTRACTS, vol. 68, no. 23, 03 juin 1968, Columbus, OH (US); T.V. FINOGE-NOVA et al., p. 9920, no. 102810k&NUM;**

**CHEMICAL ABSTRACTS, vol. 84, no. 25, 21 juin 1976, Columbus, OH (US); p. 420, no. 178212t&NUM;**

(73) Titulaire: **COMMISSARIAT A L'ENERGIE ATO-MIOUE**
**31/33, rue de la Fédération**
**F-75015 Paris Cédex 15(FR)**

(72) Inventeur: **Besnainou, Bernard**
**Bellovisto Bâtiment B2**
**F-13100 Aix en Provence(FR)**
Inventeur: **Giani, Dominique**
**5, rue de Buire**
**F-80200 Peronne(FR)**
Inventeur: **Sahut, Claire**
**Chemin du Petit Barthélémy**
**F-13090 Aix en Provence(FR)**

(74) Mandataire: **Mongrédien, André et al**
**c/o BREVATOME**
**25, rue de Ponthieu**
**F-75008 Paris (FR)**

Rank Xerox (UK) Business Services
(3.10/3.09/3.3.3)

**Description**

La présente invention a pour objet un procédé de préparation d'acide pyruvique par fermentation, en particulier par fermentation au moyen de levures appartenant au genre Candida.

L'acide pyruvique est un matériau de départ intéressant pour la préparation d'acides aminés utiles tels que le tryptophane, la tyrosine ou l'alanine. C'est un composé qui a également de l'intérêt par lui-même en tant qu'adjuvant conférant un gout acidulé.

On sait que la culture de levures telles que du genre Candida, conduit à la production d'acide pyruvique dans le milieu de culture. Cependant, la quantité d'acide pyruvique produite dans ces conditions est faible et il est difficile d'envisager cette production à une échelle industrielle en raison de ce faible rendement.

On connaît, par le brevet français FR-A-2 277 890, un procédé de production d'acide pyruvique par fermentation, qui permet d'accroître le rendement en acide pyruvique grace à l'utilisation de levures particulières qui sont des mutants du genre Candida nécessitant pour leur croissance de la thiamine et de la méthionine.

Ce procédé présente ainsi l'inconvénient de nécessiter l'utilisation de souches de levures particulières.

La présente invention a précisément pour objet un procédé de production d'acide pyruvique qui peut être mis en oeuvre à l'échelle industrielle avec un rendement élevé, qui ne nécessite pas de souches mutées et qui permet de limiter la production simultanée d'acide alpha-cétoglutarique.

Le procédé selon l'invention de production d'acide pyruvique par fermentation consiste :

1°) - à cultiver des cellules du genre Candida dans un milieu de culture comprenant une source de carbone, une source d'azote, de la thiamine et des sels minéraux, à un pH de 4,5 à 5,5, dans des conditions aérobies,

2°) - à séparer les cellules ainsi produites du milieu de croissance,

3°) - à cultiver ces cellules dans un milieu de bioconversion contenant une source de carbone et des éléments minéraux, à un pH de 3,5 à 4,5 dans des conditions aérobies, et

4°) - à extraire du milieu de culture l'acide pyruvique produit.

Dans la troisième étape de ce procédé, le choix d'un pH de 3,5 à 4,5, de préférence d'environ 4, permet de favoriser la production d'acide pyruvique au détriment de l'acide alpha-cétoglutarique.

Par ailleurs, le choix de cette gamme de pH et d'un milieu de bioconversion qui de préférence ne contient pas de thiamine et pas de fer, permet de réaliser cette culture sans augmenter la biomasse, et de convertir ainsi en produits intéressants tels que l'acide pyruvique, la majorité du carbone introduit dans le milieu de culture.

De préférence, pour obtenir un rendement en acide pyruvique intéressant, la concentration en cellules du milieu de bioconversion est d'au moins 5 g/l au début de la culture.

Le milieu de culture utilisé dans l'invention est un milieu classique permettant de produire de l'acide pyruvique. Ainsi, il contient des sources de carbone assimilable, et des sels inorganiques.

Les sources de carbone peuvent être les saccharides tels que le glucose, le fructose, le saccharose, les hydrolysats d'amidon. Pour des raisons de coût on utilise de préférence les hydrolysats d'amidon.

Les sels inorganiques généralement utilisés dans le milieu de culture sont des sels de Fe, K, Na, Mg, Mn, N, P, S, c'est-à-dire ceux nécessaires aux synthèses enzymatiques permettant une augmentation de la biomasse.

De préférence, pour favoriser la production d'acide pyruvique et éviter une croissance de la biomasse, le milieu de bioconversion ne comprend pas d'ions $Fe^{2+}$, ni de thiamine.

En effet, dans le procédé de l'invention, la culture est effectuée de façon à produire de l'acide pyruvique et non pour développer les cellules du genre Candida et il n'est donc pas nécessaire d'ajouter des ions $Fe^{2+}$ et de la thiamine qui favorise le développement de ces cellules.

A titre d'exemple de milieu de culture susceptible d'être utilisé, on peut citer les solutions aqueuses comprenant uniquement du glucose ou un hydrolysat d'amidon, du nitrate d'ammonium, du monophosphate de potassium, du sulfate de magnésium et des ions cuivre.

Selon un mode préféré de réalisation de l'invention, le procédé de production d'acide pyruvique par fermentation comprend les étapes successives suivantes :

a) - production de cellules du genre Candida par culture aérobie dans un milieu de culture comprenant au départ au moins 0,2 g/l de cellules, les sources de carbone et d'azote, les sels minéraux et la thiamine nécessaires pour le développement des cellules, à un pH de 4,5 à 5,5, jusqu'à l'obtention d'une concentration en cellules dans le milieu de culture d'au moins 5 g/l,

b) - séparation et lavage des cellules produites dans l'étape a),

c) - culture aérobie des cellules séparées dans l'étape b) dans un milieu de bioconversion comprenant une source de carbone et des sels minéraux, mais ne contenant pas de thiamine ni de fer, à un pH de 3,5 à 4,5, avec une concentration en cellules du milieu de culture au début de la culture d'au moins 5 g/l, et

d) - extraction de l'acide pyruvique du milieu de culture.

Dans ce mode de réalisation préféré, la première étape consiste à développer les cellules du genre Candida pour obtenir la concentration voulue en cellules en utilisant un milieu de culture et un pH (d'environ 5) favorisant la croissance de ces cellules. Lorsqu'on obtient la quantité voulue de cellules, on sépare celles-ci du milieu de culture et on les lave par un milieu de culture tel que celui qui sera utilisé dans l'étape suivante c) ; dans cette étape, le choix des conditions de culture est orienté vers la production d'acide pyruvique. Ainsi, le pH est de 3,5 à 4,5, de préférence 4, et on utilise un milieu de bioconversion comprenant du carbone, mais pas de thiamine ni de fer. Lors de cette étape, l'acide pyruvique s'accumule dans le milieu de bioconversion et on l'extrait de celui-ci par des procédés classiques, par exemple en séparant l'acide pyruvique des cellules par ultrafiltration, puis l'acide pyruvique de l'acide alpha-cétoglutarique par mise en contact avec une résine échangeuse d'ions.

Généralement, on met en oeuvre ce procédé en continu dans une installation comportant un fermenteur couplé à une cellule d'ultrafiltration. Ainsi, après croissance des cellules dans la première étape, on peut réaliser la séparation des cellules du milieu de culture dans la cellule d'ultrafiltration et réaliser également le lavage des cellules séparées en changeant graduellement le milieu de culture par diafiltration. On réalise ensuite la culture, à un pH de 3,5 à 4,5, en faisant circuler le milieu de culture contenant les cellules entre le fermenteur et la cellule d'ultrafiltration pour extraire en continu l'acide pyruvique produit. L'acide pyruvique extrait dans la cellule d'ultrafiltration avec le milieu de culture peut être séparé sur une résine échangeuse d'ions.

Dans ce mode préféré de réalisation de l'invention, le milieu de culture utilisé dans l'étape a) contient des sources de carbone et d'azote ainsi que des sels minéraux. Les sources de carbone et d'azote et les sels minéraux utilisés peuvent être ceux mentionnés précédemment. Les sources d'azote peuvent être des substances organiques ou inorganiques par exemple du sulfate d'ammonium, du nitrate d'ammonium, de l'urée, de la peptone ou tout autre composé peptidique. Entre deux souches conduisant à des rendements identiques de bioconversion du substrat en acide pyruvique, des raisons de coût de la source d'azote conduisent à sélectionner la souche se contentant d'azote minéral. Dans ce cas, le milieu de culture comprend également de la thiamine qui peut être introduite sous forme pure ou sous forme de substance analogue ou d'une substance la contenant, par exemple de l'extrait de levure ou de la liqueur de macération du maïs.

Certains milieux de culture nécessitent l'apport d'autres additifs tels que vitamines, méthionine, etc. mais l'utilisation de tels additifs conduit à l'obtention de l'acide pyruvique à un coût plus élevé.

Dans les étapes a) et c) du procédé de l'invention, les conditions aérobies sont obtenues par injection d'oxygène dans le milieu de culture. Dans la première étape qui est orientée vers le développement de la biomasse, on utilise une quantité d'oxygène plus importante que dans la troisième étape c) orientée vers la production d'acide pyruvique. Dans les deux cas, on soumet le milieu de culture à une agitation.

D'autres caractéristiques et avantages de l'invention apparaîtront mieux à la lecture de la description qui suit en référence au dessin annexé sur lequel :

- la figure 1 représente de façon schématique une installation pour la mise en oeuvre du procédé de l'invention,
- la figure 2 est un diagramme illustrant les variations de la quantité d'acide pyruvique produit (courbe I), de la quantité d'acide alpha-cétoglutarique produit (courbe II), et de l'évolution de la concentration en biomasse (courbe III) en fonction de la durée de culture lorsqu'on réalise celle-ci avec un changement du pH de culture de 5 à 4 au bout de 90 h, et
- la figure 3 est un diagramme représentant la production d'acide pyruvique (courbe IV) et d'acide alpha-cétoglutarique (courbe V) par g de glucose consommé, en fonction du pH du milieu de bioconversion.

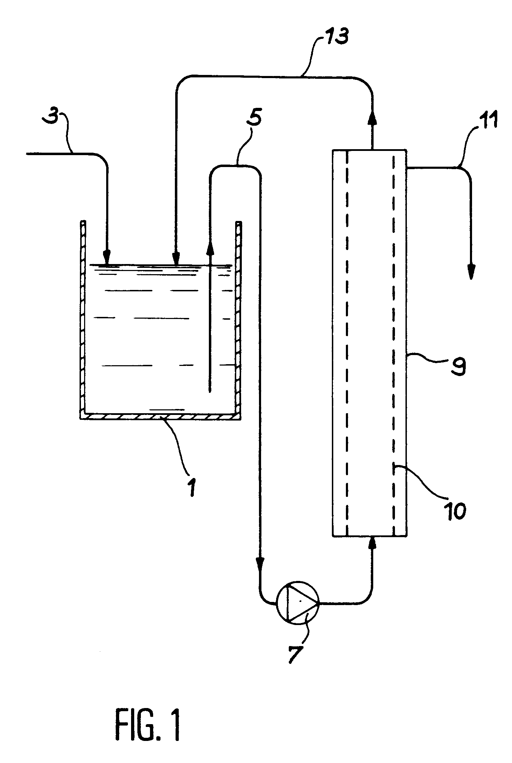

Sur la figure 1, on voit que l'installation permettant de mettre en oeuvre le procédé de l'invention comprend un fermenteur 1 muni de moyens d'alimentation 3 en produits nécessaires à la fermentation, de moyens non représentés sur le dessin de régulation de température, de régulation de pH, d'amenée d'oxygène et d'agitation.

Le milieu présent dans le fermenteur 1 peut être aspiré par la conduite 5 munie d'une pompe 7 pour être mis en circulation dans un dispositif d'ultrafiltration 9 muni de parois filtrantes 10 duquel on extrait par la conduite 11 le milieu liquide ultrafiltré qui comprend en particulier l'acide pyruvique et l'acide alpha-cétoglutarique produits lors de la fermentation. Le milieu de culture enrichi en cellules sortant du dispositif d'ultrafiltration 9 est recyclé par la conduite 13 dans le fermenteur 1.

Pour mettre en oeuvre le procédé de production d'acide pyruvique, conforme à l'invention, on opère de la façon suivante :

Etape a) : production de cellules

On réalise cette première étape en introduisant dans le fermenteur un milieu de culture C1 ayant la composition suivante :

| | |
|---|---|
| - glucose | 50 g/l, |
| - $NH_4NO_3$ | 2,5 g/l, |
| - $MgSO_4$ | 1 g/l, |
| - $KH_2PO_4$ | 2 g/l, |
| - $Fe^{2+}$ | $2.10^{-6}$ g/l, |
| - $Cu^{2+}$ | $2.10^{-6}$ g/l, |
| - extrait de viande | 0,2 g/l, |
| - thiamine | $10^{-6}$ g/l, |

auquel on ajoute 0,2 g/l de cellules du genre Candida Lipolytica.

On réalise ensuite la culture dans les conditions suivantes :
- pH : 5,
- Température : 30°C ± 3°C,
- pression partielle d'oxygène dissous : 80% ± 10% de la pression de saturation de l'oxygène dans le milieu de culture à 30°C,
- agitation : 250 à 950 tours/min (pour un Reynolds de 8500 à 32 000).

On poursuit la culture pendant 90 h en maintenant sensiblement constantes les conditions de culture.

En fin d'opération, on détermine les concentrations en cellules, en acide pyruvique et en acide alpha-cétoglutarique du milieu de culture. Les résultats obtenus sont les suivants :

| | |
|---|---|
| - cellules | 5 à 15 g/l, |
| - acide pyruvique | 10 à 15 g/l, |
| - acide alpha-cétoglutarique | 5 à 8 g/l. |

Etape b) : Séparation des cellules par ultrafiltration, lavage des cellules et changement de milieu de culture par diafiltration

Pour réaliser cette étape, on met en marche la pompe 7 et on fait circuler le milieu de culture contenant les cellules dans le dispositif d'ultrafiltration 9 qui comprend des membranes filtrantes de la marque Carbosep, constituée d'un support en carbone poreux recouvert d'une couche microporeuse d'oxyde de zirconium.

On fait circuler le milieu à une vitesse tangentielle de 4 ± 2 m/s. Lors de ce passage dans le dispositif d'ultrafiltration, le milieu s'appauvrit en liquide. Dans une première phase, on concentre les cellules de Candida Lipolytica dans 1/4 du volume initial V du milieu de culture.

Dans une deuxième phase, on introduit dans le dispositif d'ultrafiltration par la conduite 3 le milieu de culture C2 qui sera utilisé pour l'étape suivante de production d'acide pyruvique et qui présente la composition suivante :

| | |
|---|---|
| - glucose | 100 g/l, |
| - $NH_4NO_3$ | 5 g/l, |
| - $KH_2PO_4$ | 2 g/l, |
| - $MgSO_4$ | 1 g/l |
| - $Cu^{2+}$ | $2.10^{-6}$ g/l, |

4

pour ramener le volume du milieu de culture de V/4 à V/2. On interrompt alors cette addition, et on concentre à nouveau le milieu de culture de V/2 à V/4 en soutirant du liquide par la conduite 11. On renouvelle une fois ces opérations d'adjonction du second milieu de culture C2 pour passer de V/4 à V/2. Dans une dernière phase, on ajoute du second milieu de culture C2 pour amener le volume du milieu de culture circulant dans le fermenteur de V/4 à V. Ce milieu contient alors 5 à 10 g/l de cellules et on peut alors réaliser la troisième étape c).

Etape c) : Production d'acide pyruvique

On poursuit la culture des cellules dans les conditions suivantes :
- pH : 4,
- température : 30°C ± 3°C,
- Pression partielle d'oxygène : 50%,
- Agitation : 700 t/min (pour un Reynolds de 23 500),
et on maintient sensiblement constantes pendant toute la durée de culture, les conditions de culture et la composition du milieu de culture.

Pendant cette étape de production d'acide pyruvique, le milieu de culture passe dans le dispositif d'ultrafiltration et l'on extrait en continu par la conduite 11 du liquide contenant de l'acide pyruvique, de l'acide alpha-cétoglutarique et d'autres produits. On récupère l'acide pyruvique présent dans ce liquide par mise en contact avec une résine échangeuse d'ions du type ammonium quaternaire telle que la résine Duolite A162, et on recycle le liquide et les cellules par la conduite 13 dans le fermenteur 1.

Après 90 h de culture, on détermine les concentrations en cellules, en acide pyruvique et en acide alpha-cétoglutarique du milieu de culture. A titre d'exemple, un des résultats obtenus est le suivant :

| - acide pyruvique | 40,63 g/l, |
| - acide alpha-cétoglutarique | 5,90 g/l, |
| - cellules | 6,0 g/l. |

On remarque ainsi que le fait d'utiliser dans cette étape c) un pH de 4 au lieu d'un pH de 5 et un milieu de culture ne comprenant pas de thiamine, pas d'ions $Fe^{2+}$ et pas d'extrait de viande permet d'accroître la production d'acide pyruvique en diminuant la production d'acide alpha-acétoglutarique, et de maintenir la quantité de cellules du milieu de culture à une valeur pratiquement constante.

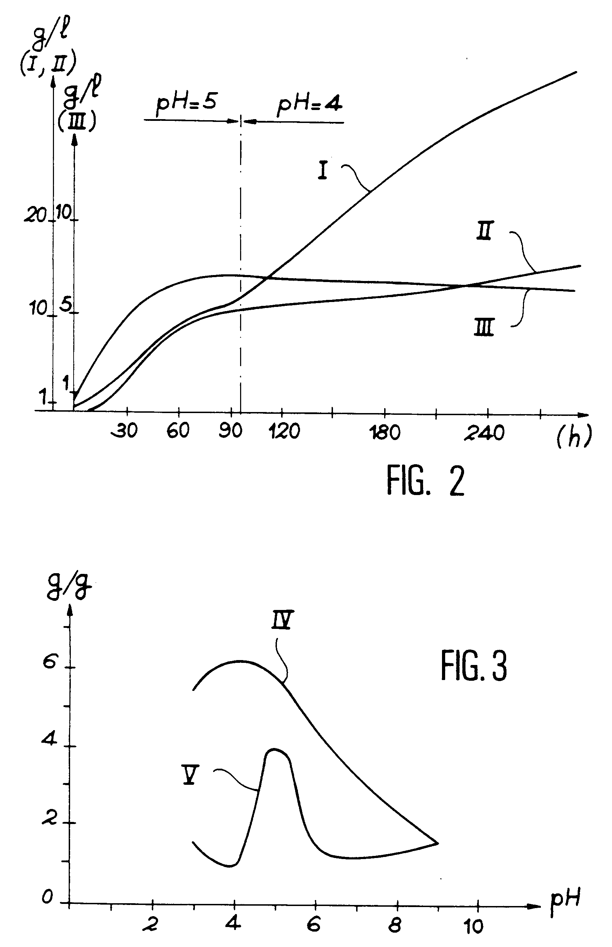

Sur la figure 2, on a représenté les variations de concentration (en g/l) d'acide pyruvique (courbe I), d'acide alpha-cétoglutarique (courbe II), et de cellules (courbe III) du milieu de culture en fonction de la durée de culture (en heures) effectuée dans les conditions décrites précédemment avec changement de milieu et de pH au bout de 90 h.

Sur cette figure, on voit que la production d'acide pyruvique et d'acide alpha-cétoglutarique est sensiblement la même au début de la culture, soit dans l'étape a) à pH 5, et qu'à partir du changement de pH et de milieu, c'est-à-dire dans l'étape c) à pH 4, la production d'acide pyruvique augmente beaucoup plus que la production d'acide alpha-cétoglutarique. La production de cellules de Candida augmente dans l'étape a) jusqu'au changement de pH et de milieu,et elle devient à peu près constante à partir de ce changement de milieu.

Ainsi, le procédé de l'invention permet d'accroître la production d'acide pyruvique par rapport à celle d'acide alpha-cétoglutarique, et il est de ce fait très intéressant pour une exploitation à l'échelle industrielle.

Dans le tableau qui suit, on a représenté le bilan en carbone lors de la production de cellules (étape a) et lors de la production d'acide pyruvique (étape c).

TABLEAU

| | BILAN EN C (g) | |
|---|---|---|
| | ETAPE a) | Etape c) |
| GLUCOSE | + 20 | + 40 |
| Cellules<br>Acide Pyruvique<br>Acide alpha-cétoglutarique<br>$CO_2$ (et autres) | 3<br>6,2<br>2,6<br>8,2 | 0<br>16,4<br>2<br>21,6 |
| | 20 | 40 |

Au vu de ces résultats, on constate que, bien qu'ayant deux fois plus de carbone dans l'étape c), la production d'acide alpha-cétoglutarique est plus faible que dans l'étape a). En revanche, le rendement de bioconversion en acide pyruvique est nettement plus important dans l'étape c) que dans l'étape a).

Dans une autre série d'expériences, on a utilisé le milieu de culture de l'étape c) et on a réalisé une bioconversion dans les mêmes conditions que celles décrites précédemment pour l'étape c) pendant 90h, mais en utilisant des pH allant de 3 à 9. En fin d'opération, on a déterminé à chaque fois les concentrations en acide pyruvique et en acide alpha-cétoglutarique du milieu de culture.

La figure 3 illustre les résultats obtenus. Sur cette figure, on a porté en ordonnée, les quantités d'acide pyruvique produites (en gramme par gramme de glucose introduit) et les quantités d'acide alpha-cétoglutarique produites (en gramme par gramme de glucose introduit). La courbe IV se rapporte à l'acide pyruvique et la courbe V à l'acide alpha-cétoglutarique.

Sur cette figure, on voit que la production d'acide pyruvique passe par un maximum à pH 4. En revanche, la courbe V qui se rapporte à la production d'acide alpha-cétoglutarique passe par un maximum très prononcé à un pH de 5 et a un minimum à pH 4.

Ainsi, l'utilisation dans l'invention d'un pH égal à 4 pour la production d'acide pyruvique permet de limiter la production d'acide alpha-cétoglutarique. En revanche, pour la ` production de la biomasse de cellules, ce qui correspond à l'étape a) du procédé de l'invention, il est préférable de se placer à un pH d'environ 5.

**Revendications**

1.  Procédé de production d'acide pyruvique par fermentation, caractérisé en ce qu'il consiste :
    1°) - à cultiver des cellules du genre Candida dans un milieu de culture comprenant une source de carbone, une source d'azote, de la thiamine et des sels minéraux, à un pH de 4,5 à 5,5, dans des conditions aérobies,
    2°) - à séparer les cellules ainsi produites du milieu de croissance,
    3°) - à cultiver ces cellules dans un milieu de bioconversion contenant une source de carbone et des éléments minéraux, à un pH de 3,5 à 4,5, dans des conditions aérobies, et
    4°) - à extraire du milieu de culture l'acide pyruvique produit.

2.  Procédé suivant la revendication 1, caractérisé en ce que le milieu de bioconversion ne comprend pas de thiamine et pas de fer.

3.  Procédé selon l'une quelconque des revendications 1 et 2, caractérisé en ce que l'on réalise la bioconversion à un pH d'environ 4.

4.  Procédé selon l'une quelconque des revendications 1 à 3, caractérisé en ce que la concentration en cellules du milieu de bioconversion, au début de la culture, est d'au moins 5 g/l.

5.  Procédé de production d'acide pyruvique par fermentation selon l'une quelconque des revendications 1 à 4, caractérisé en ce qu'il comprend les étapes successives suivantes :
    a) - production de cellules du genre Candida par culture aérobie dans un milieu de culture comprenant au départ au moins 0,2 g/l de cellules, les sources de carbone et d'azote, les sels minéraux et la thiamine nécessaires pour le développement des cellules, à un pH de 4,5 à 5,5, jusqu'à l'obtention d'une concentration en cellules dans le milieu de culture d'au moins 5 g/l,
    b) - séparation et lavage des cellules produites dans l'étape a),

6

c) - culture aérobie des cellules séparées dans l'étape b) dans un milieu de culture comprenant une source de carbone et des sels minéraux, mais ne contenant pas de thiamine ni de fer, à un pH de 3,5 à 4,5, avec une concentration en cellules du milieu de bioconversion au début de la culture d'au moins 5 g/l, et

d) - extraction de l'acide pyruvique du milieu de culture.

6. Procédé selon la revendication 5, caractérisé en ce que le pH est d'environ 5 dans la première étape a).

7. Procédé selon l'une quelconque des revendications 5 et 6, caractérisé en ce que le pH est d'environ 4 dans l'étape c).

8. Procédé selon l'une quelconque des revendications 5 à 7, caractérisé en ce que l'on réalise la séparation et le lavage des cellules produites dans l'étape a) par ultrafiltration et diafiltration.

9. Procédé selon la revendication 8, caractérisé en ce que le milieu de lavage est le milieu de culture utilisé dans l'étape c).

10. Procédé selon l'une quelconque des revendications 5 à 9, caractérisé en ce que l'on sépare l'acide pyruvique des cellules par ultrafiltration, puis l'acide pyruvique de l'acide alpha-cétoglutarique par mise en contact avec une résine échangeuse d'ions.

**Claims**

1. Process for the production of pyruvic acid by fermentation, characterized in that it comprises:
   1) culturing Candida cells in a culture medium comprising a carbon source, a nitrogen source, thiamine and mineral salts, at a pH of 4.5 to 5.5 and under aerobic conditions,
   2) separating the thus produced cells from the growth medium,
   3) culturing these cells in a bioconversion medium containing a carbon source and mineral elements, at a pH of 3.5 to 4.5 and under aerobic conditions, and
   4) extracting the pyruvic acid produced from the culture medium.

2. Process according to claim 1, characterized in that the bioconversion medium comprises no thiamine or iron.

3. Process according to either of the claims 1 and 2, characterized in that bioconversion is carried out at a pH of approximately 4.

4. Process according to any one of the claims 1 to 3, characterized in that the cell concentration of the bioconversion medium at the start of culturing is at least 5 g/l.

5. Process for the production of pyruvic acid by fermentation according to any one of the claims 1 to 4, characterized in that it comprises the following stages:
   a) production of Candida cells by aerobic culturing in a culture medium initially containing at least 0.2 g/l of cells, the carbon and nitrogen sources, the mineral salts and the thiamine necessary for the development of the cells, at a pH of 4.5 to 5.5, until a cell concentration in the culture medium of at least 5 g/l is obtained,
   b) separation and washing of the cells produced in stage a),
   c) aerobic culturing of the cells separated in stage b) in a culture medium containing a carbon source and mineral salts, but containing no thiamine or iron, at a ph of 3.5 to 4.5 and with a cell concentration in the bioconversion medium at the start of culturing of at least 5 g/l, and
   d) extraction of the pyruvic acid from the culture medium.

6. Process according to claim 5, characterized in that the pH is approximately 5 in the first stage a).

7. Process according to either of the claims 5 and 6, characterized in that the pH is approximately 4 in stage c).

**8.** Process according to any one of the claims 5 to 7, characterized in that the separation and washing of the cells produced in stage a) takes place by ultrafiltration and diafiltration.

**9.** Process according to claim 8, characterized in that the washing medium is the culture medium used in stage c).

**10.** Process according to any one of the claims 5 to 9, characterized in that the pyruvic acid is separated from the cells by ultrafiltration, and then the pyruvic acid is separated from the alpha-ketoglutaric acid by contacting with an ion exchange resin.

**Patentansprüche**

**1.** Verfahren zur Herstellung von Brenztraubensäure durch Fermentation, dadurch gekennzeichnet, daß es darin besteht,
1°) - Zellen der Gattung Candida in einem Kulturmedium, das eine Quelle für Kohlenstoff, eine Quelle für Stickstoff, Thiamin und Mineralsalze enthält, bei einem pH von 4,5 bis 5,5 unter aeroben Bedingungen zu kultivieren,
2°) - die so erzeugten Zellen vom Kulturmedium zu trennen,
3°) - diese Zellen im einem Medium zur Biokonversion, das eine Quelle für Kohlenstoff und mineralische Elemente enthält, bei einem pH von 3,5 bis 4,5 unter aeroben Bedingungen zu kultivieren und
4°) - die erzeugte Brenztraubensäure vom Kulturmedium zu extrahieren.

**2.** Verfahren nach Anspruch 1, dadurch gekennzeichnet, daß das Medium zur Biokonversion kein Thiamin und kein Eisen enthält.

**3.** Verfahren nach einem dem Ansprüche 1 und 2, dadurch gekennzeichnet, daß die Biokonversion bei einem pH von ungefähr 4 durchgeführt wird.

**4.** Verfahren nach einem der Ansprüche 1 bis 3, dadurch gekennzeichnet, daß die Zellkonzentration des Biokonversionsmediums zu Beginn der Kultur mindestens 5 g/l ist.

**5.** Verfahren zur Herstellung von Brenztraubensäure durch Fermentation nach einem der Ansprüche 1 bis 4, dadurch gekennzeichnet, daß es nacheinander die folgenden Schritte umfaßt:
a) - Erzeugung von Zellen der Gattung Candida durch aerobe Kultur in einem Kulturmedium, das zu Beginn wenigstens 0,2 g/l Zellen, die Quellen für Kohlenstoff und für Stickstoff, die für die Entwicklung der Zellen nötigen Mineralsalze und Thiamin enthält, bei einem pH von 4,5 bis 5,5 bis zum Erhalt einer Zellkonzentration im Kulturmedium von wenigstens 5 g/l,
b) - Abtrennen und Waschen der in Schritt a) erzeugten Zellen,
c) - aerobe Kultur der in Schritt b) abgetrennten Zellen in einem Kulturmedium, das eine Quelle für Kohlenstoff und Mineralsalze umfaßt, das aber weder Thiamin noch Eisen enthält, bei einem pH von 3,5 bis 4,5 mit einer Zellkonzentration im Biokonversionsmedium zu Beginn der Kultur von mindestens 5 g/l und
d) - Extrahieren der Brenztraubensäure vom Kulturmedium.

**6.** Verfahren nach Anspruch 5, dadurch gekennzeichnet, daß der pH im ersten Schritt a) ungefähr 5 ist.

**7.** Verfahren nach einem der Ansprüche 5 und 6, dadurch gekennzeichnet, daß der pH im Schritt c) ungefähr 4 ist.

**8.** Verfahren nach einem der Ansprüche 5 bis 7, dadurch gekennzeichnet, daß das Abtrennen und das Waschen der in Schritt a) erzeugten Zellen durch Ultrafiltration und Diafiltration geschieht.

**9.** Verfahren nach Anspruch 8, dadurch gekennzeichnet, daß das Waschmedium das im Schritt c) verwendete Kulturmedium ist.

**10.** Verfahren nach einem der Ansprüche 5 bis 9, dadurch gekennzeichnet, daß die Brenztraubensäure von den Zellen durch Ultrafiltration abgetrennt wird und anschließend die Brenztraubensäure von der $\alpha$-Ketoglutarsäure durch Zusammenbringen mit einem Ionenaustauscherharz abgetrennt wird.

FIG. 1

FIG. 2

FIG. 3